# EUROPEAN PATENT APPLICATION

(11) **EP 0 861 891 A1**
(43) Date of publication of application: **02.09.1998**
(21) Application number: 96402930.0
(22) Date of filing: 31.12.1996
(51) Int. Cl.: C12N 15/12, A61K 39/395, C12N 5/10, G01N 33/53

(54) **Compounds for modulating cell negative regulations and biological applications thereof**

(71) Applicant: INSTITUT NATIONAL DE LA SANTE ET DE LA RECHERCHE MEDICALE (INSERM), 75654 Paris Cédex 13 (FR)
(72) Inventor: Daeron, Marc, 78460 Chevreuse (FR); Vivier, Eric, 13260 Cassis (FR)
(74) Representative: Peaucelle, Chantal

(57) **Abstract**

The invention relates to compounds for modulating cell negative regulations and biological applications thereof.

It particularly relates to compounds capable of cross-linking a stimulatory receptor with a KIR (Killer-cell inhibitory receptor) or with an homologue thereof.

## Description

The invention relates to compounds capable of modulating cell negative regulations. It also relates to the biological applications of said compounds. Cell negative regulations dysfunctions can lead to diseases such as allergic, inflammatory or cytotoxicity-related diseases.

Cytotoxicity is a major strategy used by the immune system to eliminate cellular antigens such as virus-infected cells and tumor cells.

NK (Natural Killer cells) cells are spontaneously cytotoxic lymphocytes, capable of recognizing antigens expressed by tumoral cells.

NK cells are also involved in autoimmune, immunoproliferative and immunodeficiency diseases.

NK cells can induce the lysis of target cells by two mechanisms. Antibody-dependent cell cytotoxicity (ADCC) leads to the lysis of antibody-coated target cells, whereas natural cytotoxicity leads to the antibody-independent lysis of a variety of cell targets, including primarily virus-infected cells and tumor cells.

NK cells represent a peculiar class of lymphocytes which cannot rearrange antigen receptor gene segments. NK cells are however capable of recognizing and inducing the lysis of deleterious cells, and primarily *in vitro* tumor cells as well as virus-infected cells.

A major mechanism which controls NK cell cytotoxic function is initiated by the recognition of MHC Class I molecules expressed at the surface of target cells. NK cells express several cell surface receptors for MHC Class I molecules, *i.e.* the so called KIR (Killer-cell Inhibitory Receptors).

In contrast to the T cell receptor complex (CD3/TCR), KIR are characterized (i) by their ability to interact with a large panel of MHC Class I allele products (promiscuous recognition), and (ii) by their ability to transduce a negative signal which leads to the inhibition of both natural cytotoxicity and ADCC programs.

KIR are not NK cell-restricted since they are also expressed on T cell subsets and can inhibit T cell activation triggered *via* the CD3/TCR complexes.

Human and mouse KIR (MHC Class I inhibitory receptors) belong to two distinct families: immunoglobulin superfamily (IgSF) and C2 lectins. Lectin-like KIR are receptors for MHC Class I molecules and also for carbohydrates. In particular, human KIR IgSF include p58 (CD158), p70 (CDw159) and p140 (CDw160) molecules whereas human KIR C2 lectin include the CD94-NKG2A/B heterodimer.

KIR homologues such as gp49B1 are also in the scope of the present invention. gp49B1 is an IgSF member which present significant homologies with the extracellular, transmembrane as well as with the intracytoplasmic domains of KIR. The receptor gp49B1 is not NK cell-restricted: it is also expressed on mast cells.

KIR and KIR homologues are therefore all expressed on NK cells but none of them are NK-restricted. They can therefore not only be involved in autoimmune but also in inflammatory diseases and immunoproliferative and immunodeficiency diseases.

Research relating to cell regulation has up to now focused on cell receptors for Fc (immunoglobulin constant fragments) such as FcγRIIB, and on antigen-specific receptors of T and B cells.

The balance between receptor-mediated activation and inactivation is central to *in vivo* homeostasis.

The cell surface receptors initiating NK cell activatory pathways comprise:
i. the ADCC receptor complex, including FcγRIIIA (CD16, which is the only Fc receptor expressed on NK cells), KAR (Killer cell Activatory Receptor) and a variety of disulfide-linked hetero- and homodimers associated with CD16. The engagement of the ADCC receptor initiates a series of ITAM-dependent (Immunoreceptor Tyrosine-based Activation Motifs) signaling pathways, leading to the release of intracytoplasmic NK granules as well as the transcription of a set of genes encoding surface activation molecules (*e.g.* CD69, CD25) and cytokines (*e.g.* α-IFN),
ii. the NK receptors mediating activatory signals for the initiation of natural cytotoxicity programs, such as NKRP-1 proteins,
iii. Lag 3, a molecule expressed on activated T and NK cells which is homologous to CD4.
iv. adhesion molecules, such as the Beta-2 integrin expressed on NK cells or DNAM-1 expressed in most T and NK lymphocytes and on a subpopulation of B lymphocytes.

From the prior art teaching, four mechanisms can be essentially considered as modulating cell activation. These mechanisms could lead to
- a direct interference with the ligand-activatory receptor binding events, such has been observed in cytokine biology with the interleukin-1 receptor antagonist,
- a down-regulation of the activatory receptor membrane expression, such has been observed with the epidermal growth factor receptor,
- an interference with the effector function coupled to the activation receptor, *i.e.* an interference with the transcription of the set of genes induced by the activation cascade, such has been observed with the glucocorticoids, or
- an interference with the early signaling pathway coupled to the activation receptor, such has been observed with the heterotrimeric G-protein or with the receptors coupled to PTK activation.

The present invention herein demonstrates that KIR necessarily requires co-aggregation with activatory receptors to exert their inhibitory functions on said activatory receptors.

The present invention also demonstrates that KIR, normally expressed on NK or T cells, can function in non-lymphoid cells (on which KIR homologues are expressed) and that KIR and KIR homologues thereby can inhibit the activation of receptors involved in inflammatory and allergic responses.

The present invention therefore surprisingly demonstrates that a KIR or a KIR homologue is capable of modulating the activation of several ITAM-bearing receptors.

The present invention also gives the first report of the obtention of a specific anti-ITIM (Immunoreceptor Tyrosine-based Inhibition Motif) compound.

The present invention further demonstrates that the KIR and KIR homologue family exerts regulatory functions and uses strategies to mediate its inhibitory functions distinct and divergent from those exerted and used by other members of the ITIM-bearing receptor family.

One aspect of the invention accordingly relates to a compound capable of cross-linking a stimulatory receptor with a KIR or an homologue thereof.

In many embodiments, it will be desirable to provide a compound capable of specifically regulating the activation of a KIR or a KIR homologue and/or capable of regulating the activation of a stimulatory receptor.

Said stimulatory receptor is particularly an ITAM-bearing receptor such as KAR, FcεRI, CD3/TCR, CD16, any receptor related to tyrosine kinase activities, such as a growth factor receptor, or a receptor sub-unit such as CD3ζ, CD3ε, CD3Υ, CD3δ or FcεRIγ.

Said KIR is a IgSF member, such as CDI58, CDW159, CDW 160, or is lectin-like, such as NKG2A the CD94-CD94 homodimer or the CD94/NKG2A heterodimer and said KIR homologue is gp49B1.

Said KIR or KIR homologue may be expressed on a NK, a T or a mast cell or on a monocyte or is recombinantly expressed.

The compound of the invention is further characterized in that it is capable of inducing the regulation of free calcium concentration in a cell. Said compound is most preferably capable of inducing the regulation of calcium influx into a cell and/or of calcium mobilization from intracellular compartments.

Said compound is further characterized in that it is capable of inducing the recruitment by said KIR or KIR homologue of a phosphatase selected from the group consisting of SHP-1, SHP-2, SHIP and related phosphatases.

In preferred embodiments, said compound is essentially a polypeptide, a glycoprotein or a carbohydrate.

In other preferred embodiments, said compound is a bispecific reagent and/or a chemical inducer of dimerization. It may be produced by chemical synthesis or by genetic engineering.

In yet other preferred embodiments, said compound is a bispecific antibody. For example, said compound may comprise at least one Fab, Fd, Fv, dAb, CDR, F(ab')₂ , VH, VL, ScFv fragment.

In most preferred embodiments, said compound is capable of cross-linking said KIR or said KIR homologue with said stimulatory receptor in the extracellular domain of a cell. Said compound can advantageously cross-link a stimulatory receptor with any KIR that is Ig-like or with any KIR that is lectin-like.

In other most preferred embodiments, said compound is capable of crossing through a lipid bi-layer. For example, it may be liposoluble and/or associated with a drug-delivery system.

In yet other most preferred embodiments, said compound is capable of cross-linking said KIR or said KIR homologue with said stimulatory receptor in the intracellular domain of a cell. Said compound can advantageously cross-link a stimulatory receptor with define KIR (Ig-like or lectin-like) indiscriminately with any KIR (Ig-like and lectin-like). Said compound may be advantageously associated with a drug-delivery system.

In certain preferred embodiments, said compound is capable of modulating the release of serotonin and/or of inflammatory mediators by a cell expressing FcεRI, such as a mast cell, and/or of modulating cytokine release (Interleukin-6, Tumor Necrosis Factor Alpha release) from a cell, such as a mast cell or a NK cell, and/or of modulating interleukin production such as the IL-2 production and/or the γ-interferon production from a peripheral blood cell and/or of modulating the proliferation of peripheral blood cells.

Another aspect of the invention provides a nucleic acid coding for a polypeptide according to the invention and a cell transfected by said nucleic acid.

In still another aspect, the invention relates to a pharmaceutically acceptable preparation comprising a therapeutically-effective amount of at least one compound of the invention. Such a pharmaceutical preparation is useful for modulating an animal cell function involved in a disease selected from the group consisting of immunoproliferative diseases, immunodeficiency diseases, cancers, autoimmune diseases, infectious diseases, viral diseases, inflammatory responses, allergic responses or involved in organ transplant tolerance.

The pharmaceutical preparation of the invention may be formulated in solid or liquid form or in suspension for oral administration, parenteral administration, topical, intravaginal or intrarectal application, or for nasal and/or oral inhalation.

The present invention also makes available a method for the *in vitro* or *ex vivo* diagnosis of a cell disregulation, comprising the step of estimating of the relative proportion of co-aggregated KIR or KIR homologue *vs*. non-co-aggregated KIR or KIR homologue by:
- contacting a biological sample with a compound, or with a nucleic acid, or a cell according to the invention, and
- of revealing the reaction product possibly formed.

Estimating the relative proportion of co-aggregated KIR or KIR homologue *vs* non-co-aggregated KIR or KIR homologue is particularly useful for the precise diagnosis of diseases where cell disregulation is involved, such as immunoproliferative diseases, immunodeficiency diseases, cancers, autoimmune diseases, infectious diseases, viral diseases, inflammatory responses, allergic responses and for the choice of the appropriate treatment.

Other aspects and embodiments of the present invention will become obvious to one of ordinary skill in the art after consideration of the drawing and examples provided below.

What follows should not be interpreted as limiting the invention in any way.

### Ten figures are mentioned:

- figure 1 illustrates the reconstitution of wild-type and mutant p58.2 HLA-Cw3-specific KIR in RTIIB cells,
- figure 2 illustrates the surface receptor-induced serotonin release in RTIIB cells expressing human KIRs,
- figure 3 shows that human KIRs inhibit ITAM-dependent RTIIB cells serotonin release,
- figure 4 shows that the inhibition of ITAM-dependent RTIIB cells serotonin release requires KIR co-aggregation,
- figure 5 shows that human KIRs inhibit ITAM-dependent intracytoplasmic Ca²⁺ mobilization in RTIIB cells,
- figure 6 shows that gp 49B1 ITIMs bind the phosphatase SHP-1, as assessed by surface plasmon resonance analysis,
- figure 7 shows that gp 49B1 ITIMs bind the phosphatase SHIP, as assessed by cell lysates adsorption to synthetic peptides,
- figure 8 shows that gp49B1 ITIMs bind SHP-1, as assessed by cell lysate adsorption to synthetic peptides.
- figure 9 shows immunofluorescence and flow cytometry analysis of splenocyte and peripheral blood lymphocytes isolated from p58.2 transgenic mice and,
- figure 10 shows the effect of NK cells isolated from p58.2 transgenic mice on parental p815 cells and on P815-Cw3 cells.

### ABBREVIATIONS

- ADCCR:: Antibody-Dependent Cell Cytotoxicity Receptor complex.
- Ca²⁺ᵢ:: Intracellular Ca²⁺ concentration.
- DAM:: Donkey Anti-Mouse Ig antiserum.
- DAR:: Donkey Anti-Rat Ig antiserum.
- GAM:: Goat Anti-Mouse Ig antiserum.
- GST :: Glutathion S-transferase
- ITAM:: Immunoreceptor Tyrosine-based Activation Motif.
- ITIM:: Immonoreceptor Tyrosine-based Inhibition Motif.
- KAR:: Killer-cell Activatory Receptor.
- KIR:: Killer-cell Inhibitory Receptor.
- Kd :: Equilibrium dissociation constant
- mAb:: monoclonal Antibody
- PTK:: Protein Tyrosine Kinase.
- PTPase:: Protein Tyrosine Phosphatase.
- SHIP:: Phosphatidylinositol phosphatase
- SH2:: *src*-homology domain 2.
- SPR :: Surface plasmon resonance

### Example 1:

. **KIR can function in a non lymphoid cell line**,
. **KIR only inhibit the functions of the activatory receptors with which they are co-aggregated (serotonin release, Ca**^{**2+**} **influx and mobilization)**,
. **KIR and Fc ITIM-bearing receptors exert distinct regulatory functions**.

HLA-Cw3-specific KIR (p58.2) has been reconstituted in a transfected mast cell/basophil-like RBL-2H3 cell line: (RTIIB). RTIIB cells express two distinct ITAM-dependent receptors: the endogenous FcεRI antigen receptor and a transfected CD25/CD3ζ chimeric molecule. A naturally-occuring mutant of p58.2, the p50.2 KAR (Killer-cell Activatory Receptor), has also been reconstituted in RTIIB cells. The p50.2 KAR expresses a shorter intracytoplasmic domain, which does not contain any I/VxYxxL/V stretch (ITIM motif). This mutant receptor is able to trigger T and NK cell activation programs.

### Experimental procedures:

***Antibodies**.* The following mAb and antisera were obtained from Immunotech, Marseille, France: mouse anti-human CD25 mAb (B1.49.9, IgG2a), rat anti-human CD25 mAb (33B3.1, IgG2a), mouse anti-human p58.2 mAb (GL183, IgG1), mouse anti-rat Ig (H+L) F(ab')₂(MAR), goat anti-mouse Ig (H+L) F(a,b)₂ (GAM), donkey anti-mouse Ig (H+L) F(ab')₂ (DAM), donkey anti-rat Ig (H+L) F(ab')₂(DAR). Rat IgE mAb (LO-DNP-30), mouse IgE mAb (2682-I), rat anti-FcγRII/III mAb (2.4G2), mouse anti-CD25 mAb (7G7,IgG1) and mouse anti-rat FcεRIα (BC4,IgG1) were also used. Mouse IgE mAb was used as a dilution of hybridoma supernatants. All other mAb were used as protein A/G purified mAb. GL183, 2.4G2 and 7G7 mAb were used as F(ab')₂; LO-DNP-30, 2682-I, B1.49.9 and 33.B3.1 were used as intact mAb. LO-DNP-30 and 2682-I are directed against DNP and TNP. MAR F(ab')₂ was trinitrophenylated using trinitrobenzene sulfonic acid (Eastman-Kodak,Rochester, NY, USA), 1 mole of MAR F(ab')₂ was substituted with an average number of 10 TNP moles. This TNP-F(ab')₂MAR was used to crosslink mouse anti-TNP IgE and rat anti-FcγRII/III 2.4G2.

***Cells**.* All cells were cultured in DMEM supplemented with 10% FCS and penicillin (100 IU/ml)-streptomycin (100 µg/ml). RBL-2H3 cell transfectants expressing murine FcγRIIb2 and CD25/CD3ζ chimeric molecules (RTIIB) have been previously described. The CD25/CD3ζ chimeric molecule includes the complete human CD25 ecto- and transmembrane domains linked to the complete mouse CD3ζ intracytoplasmic domain. RTIIB cells were transfected by electroporation using either the 183.6 cDNA encoding p58.2 (RTIIB.p58) or the 183.Act1 cDNA encoding p50.2 (RTIIB.p50), in the RSV-5.gpt expression vector. Stable transfectants were established by culture in the presence of xanthine (250 µg/ml), hypoxanthine (13.6 µg/ml) and mycophenolic acid (2 µg/ml). Two representative clones of each transfection series (RTIIB.p58A, RTIIB.p58B and RTIIB.p50A, RTIIB.p50B) were examined in parallel and gave similar results. Unless indicated, results from one clone of each transfection series are shown.

**Flow cytometric analysis**. The primary mAb was incubated with cells on ice for 20-30 minutes, followed by 3 washes with PBS supplemented with 0.2% BSA. The secondary staining was performed using fluorescein-conjugated rabbit anti-mouse IgG (Immunotech, Marseille, France), followed by 3 PBS/0.2% BSA washes and resuspension in PBS containing 1% formaldehyde. Cells were analyzed on a FACS-Scan apparatus (Becton-Dickinson, Mountain View, CA, USA).

***Single cell Ca***^{***2+***} ***video-imaging**.* Cells (2 x 10⁵/sample) were allowed to adhere overnight onto glass coverslips in culture medium. Adherent cells were washed and then incubated at 37°C for 40 minutes in RPMI medium supplemented with 10% FCS with a 10⁻³ dilution of mouse IgE (2682-I) in the presence or absence of either 1 µg/ml GL183 or 1 µg/ml 2.4G2. 1 µM Fura-2/AM (Molecular Probes, Eugene, OR, USA) in dimethylsulfoxide premixed with 0.2 mg/ml pluronic acid (Molecular Probes) was then added to the medium for 20 minutes. Cells were washed and then measurements of intracytoplasmic Ca²⁺ (Ca²⁺ᵢ) mobilization were performed at 37°C in MS buffer (140 mM NaCl, 5 mM KCl, 10 mM HEPES, pH 7.4, 1 mM CaCl₂, 1 mM MgCl₂) with a Nikon Diaphot 300 microscope and an IMSTAR imaging system. Briefly, each [Ca²⁺]ᵢ image (taken every 6 seconds) was calculated from the ratio of the average of 4 fluorescence images after 340 nm excitation, and 4 fluorescence images after 380 nm excitation. Ca²⁺ᵢ values were calculated according to Grynkiewicz et al. 1985, *J.Biol. Chem.* 260:3440-3450. The stimulation was done by adding GAM F(ab')₂ or TNP-F(ab')₂MAR to the MS buffer to a final concentration of 50 µg/ml and 10 µg/ml respectively. The measurements of intracellular calcium stocks release was done by replacing the MS buffer with a stimulation buffer (150 mM NaCl, 5 mM KCl, 1 mM MgCl2, 0.5 mM EGTA, 20 mM HEPES, pH 7.3, 50 µg/ml F(ab')₂ GAM) at the time of stimulation. For each experiments, results were obtained as the average variation of Ca²⁺ᵢ (nM) as a function of time for a population of 20-30 cells.

***Serotonin release**.* RTIIB cell transfectants harvested using trypsin-EDTA were examined for serotonin release. Briefly, cells were resuspended in RPMI medium supplemented with 10% FCS at 1 x 10⁶ cells/ml, and incubated at 37°C for 1 h with 2 µCi/ml [³H] serotonin (Amersham, Les Ulis, France), washed, resuspended in RPMI-10% FCS, incubated for another hour at 37°C, washed again, resuspended in the same medium and distributed in 96-well microculture plates at 2 x 10⁵ cells/well. Cells were then incubated for 1 h with mouse or rat IgE, with mouse or rat anti-CD25 mAb in the absence or in the presence of GL183 F(ab')₂, in a final volume of 50 µl. Cells were washed 3 times in 200 µl PBS and once in 200 µl RPMI-10% FCS, then 25 µl culture medium were added to each well and cells were warmed for 15 minutes at 37°C before challenge. Cells were challenged for 30 minutes at 37°C with 25 µl prewarmed GAM, DAM or DAR F(ab')₂ as indicated. Reactions were stopped by adding 50 µl ice-cold medium and by placing plates on ice. 50 µl of supernatants were mixed with 1 ml Emulsifier Safe scintillation liquid (Packard, Groningen, The Netherlands) and counted in a LS6000 Beckman counter. The percentage of serotonin release was calculated using as 100%, cpm contained in 50 µl harvested from wells containing the same number of cells and lysed in 100 µl 0,5% SDS-0.5% NP40.

### Results

***Reconstitution of human KIR and KAR in RTIIB cells****.* Stable RTIIB cell transfectants expressing murine FcγRIIB as well as a CD25/CD3ζ chimeric molecule at their surface, were further transfected with two distinct NK cell MHC Class I receptor p58.2 and p50.2 cDNA constructions. In NK cells, the wild type p58.2 (KIR) exerts an inhibitory effect whereas the mutant p50.2 (KAR) is an activating molecule. Despite this striking difference, both p58.2 and p50.2 are HLA-Cw3-specific receptors and are recognized by the GL183 mAb. Representative transfected clones used thereafter were selected for their matched cell surface expression of the wild-type p58.2 (RTIIB.p58) or the mutant p50.2 (RTIIB.p50) HLA-Cw3 receptors.

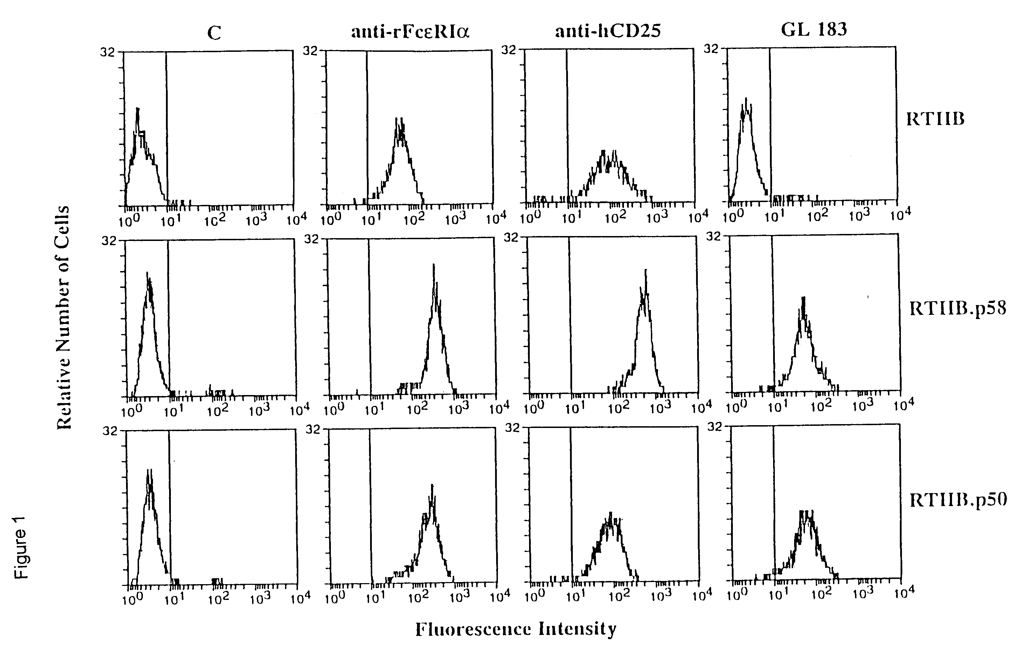

Figure 1 illustrates the reconstitution of wild-type and mutant p58.2 HLA-Cw3-specific KIR in RTIIB cells, where representative transfected RTIIB clones were stained by indirect immunofluorescence using mAb directed against rat FcεRI (BC4), human CD25 (B1.49.9), as well as human wild-type p58.2 KIR and mutant p50.2 KAR (GL183). Negative controls were only incubated with fluorescein conjugated rabbit anti-mouse IgG also used as secondary reagents.

***Inhibition of ITAM-dependent serotonin release by KIR reconstituted in RTIIB cells**.* RTIIB cells can be induced to release serotonin by one of two ways: aggregation of the endogenous rat FcεRI receptor complex or aggregation of the CD25/CD3ζ chimeric molecule. Mouse IgE binding to FcεRI is not sufficient to induce RTIIB serotonin release, and aggregation of FcεRI receptors was obtained using GAM F(ab')₂. The integrity of ITAM expressed by both receptors is required for RTIIB serotonin release, indicating that both FcεRI- and CD25/CD3ζ-induced serotonin release utilize ITAM-dependent signaling mechanism. Using RTIIB cells expressing p58.2 KIR or the mutant p50.2 KAR in addition to FcεRI and CD25/CD3ζ, the expression or the aggregation of reconstituted HLA-Cw3 receptors, was investigated with respect to ITAM-dependent serotonin release modulation.

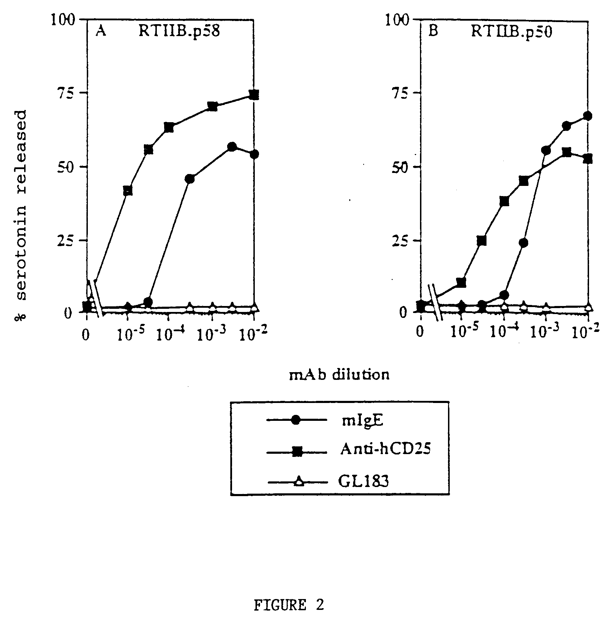

Figure 2 illustrates the surface receptor-induced serotonin release in RTIIB cells expressing human KIR:
RTIIB.p58 cells (**A**) or RTIIB.p50 cells (**B**) were incubated 1 h at 37°C either with serial dilution of mouse IgE (2682-I, initial concentration: straight hybridoma supernatant) (closed circles), anti-hCD25 (F(ab')₂ 7G7, initial concentration: 1 mg/ml) (closed squares) or GL183 F(ab')₂ (initial concentration: 1 mg/ml) (open triangles). After being washed, cells were challenged for 30 minutes at 37°C with 50 µg/ml GAM F(ab')₂. The serotonin released in supernatants was measured. The experiment shown is representative of three independent experiments.

As shown in Fig. 2A and B, aggregation of FcεRI or CD25/CD3ζ receptors induces a dose-dependent serotonin release of RTIIB cells expressing either p58.2 KIR (RTIIB.p58) or p50.2 KAR (RTIIB.p50). The larger serotonin release elicited by anti-CD25 in RTIIB.p58 compared to RTIIB.p50 cells most likely reflects the difference in surface expression of CD25/CD3ζ in the two cell types. When p58.2 KIR were aggregated using anti- p58.2 mAb (GL183), no RTIIB serotonin release was observed. This result is consistent with the lack of detectable signals induced in NK and T cells upon anti-p58.2 mAb stimulation. However, no serotonin release was detected in response to GL183 mAb in RTIIB.p50 cells, in contrast with the stimulatory effect of p50.2 KAR reported in both NK and T cells.

In a second set of experiments, p58.2 KIR *vs*. p50.2 KAR and FcεRI receptors were co-aggregated using mouse IgE, mouse GL183 and GAM.

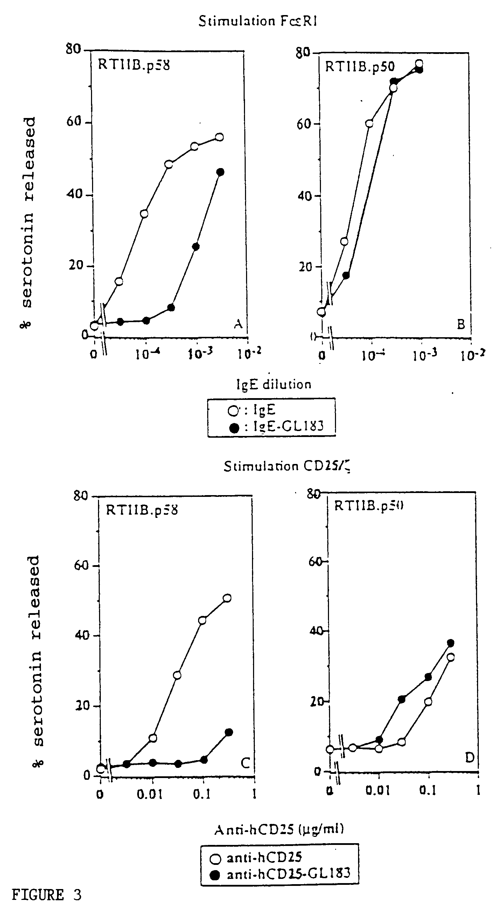

Results are reported in figure 3: RTIIB.p58 cells (**A**,**C**) and RTIIB.p50 cells (**B**,**D**) were incubated 1 h at 37°C with 3 µg/ml GL183 F(ab')₂ and mouse IgE (2682-I) (A and B, closed circles) or with 3 µg/ml GL183 F(ab')₂ and anti-hCD25 (F(ab')₂ 7G7) (C and D, closed circles). Controls were made without GL183 F(ab')₂ (open squares). After being washed, cells were challenged for 30 minutes at 37°C with 50 µg/ml GAM F(ab')₂. The serotonin released in supernatants was measured. The experiment shown is representative of three independent experiments.

Using saturating concentrations of GL183, the serotonin release induced by FcεRI was impaired in RTIIB.p58 cells (Fig. 3A). This inhibition was detectable at sub-optimal (< 10⁻³ dilution), as well as optimal concentration of IgE (10⁻³ dilution). Using saturating concentrations of both GL183 (1 µg/ml) and IgE (10⁻³ dilution), 72.8% ± 2.2 and 55.8% ± 9.9 (mean ± 1 SEM, n=3) inhibition of serotonin release were observed in two distinct RTIIB.p58 clones. Similar results were obtained when serotonin release was triggered *via* the CD25/CD3ζ chimeric molecule (Fig. 3C). Using saturating concentrations of both GL183 (1 µg/ml) and anti-hCD25 ( 3 µg/ml), serotonin release was inhibited by 39.3% ± 11.9 in the representative RTIIB.p58B clone.

In contrast, no significant inhibition or potentiation of ITAM-dependent cell activation was detected when p50.2 KAR was co-aggregated with either FcεRI or CD25/CD3ζ surface receptors, even at sub-optimal concentration of triggering IgE or anti-hCD25, in the presence of saturating concentration of GL183 (1 µg/ml)(Fig. 3B and 3D).

These results first demonstrate that p58.2 KIR reconstituted in RTIIB cells are functional, and inhibit ITAM-dependent cell activation. Second, they show that the integrity of p58.2 intracytoplasmic sequence is required for KIR-mediated inhibition of RTIIB serotonin release. Finally, these data indicate that RTIIB cells provide an appropriate cellular environment for a functional reconstitution of p58.2 KIR, but not of p50.2 KAR.

***KIR inhibitory function requires co-engagement of KIR and ITAM-containing receptors**.* In a next set of experiments, RTIIB.p58 cells were stimulated *via* FcεRI in the presence of aggregated p58.2 in one of two ways.

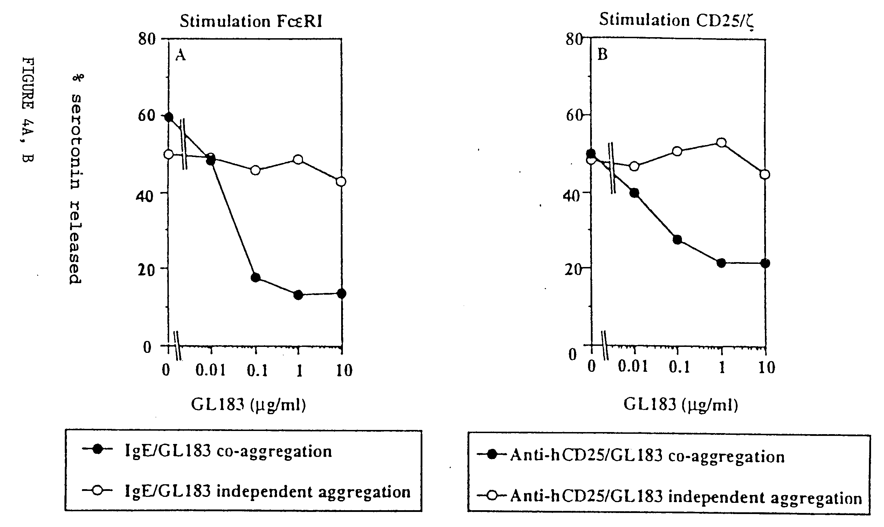

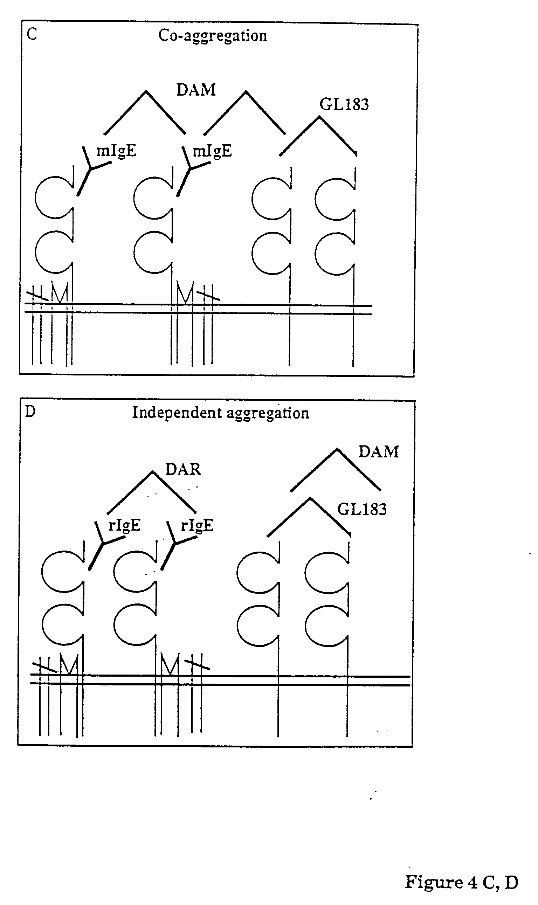

Results are reported in Figure 4:
(**A**) RTIIB.p58 cells were incubated 1 h at 37°C with indicated concentrations of GL183 F(ab')₂ and either 10⁻³ mIgE (2682-I) dilution (closed circles) or 10⁻³rIgE (LO-DNP-30,1 mg/ml initial concentration) dilution (open circles). After being washed, cells were challenged for 30 minutes at 37°C with 50 µg/ml DAM F(ab')₂ (closed circles) or with 50 µg/ml DAM F(ab')₂ plus 50 µg/ml DAR F(ab')₂ (open circles). The serotonin released in supernatants was measured.
(**B**) RTIIB.p58 cells were incubated 1 h at 37°C with indicated concentrations of GL183 F(ab')₂ and either 3 µg/ml anti-hCD25 mAb (mB1.49.9) (closed circles) or 3 µg/ml anti-hCD25 mAb (r33B3.1) (open circles). After being washed, cells were challenged for 30 minutes at 37°c with 50 µg/ml DAM F(ab')₂ (closed circles) or with 50 µg/ml DAM F(ab')₂ plus 50 µg/ml DAR F(ab')₂ (open circles). The serotonin released in supernatants was measured.
   This experiment is representative of five independent experiments.
   Co-aggregation and independent aggregation experiments are schematized in (**C**) and (**D**) respectively.

DAM was used to co-aggregate FcεRI and p58.2 KIR *via* mouse IgE and mouse GL183 respectively (Fig. 4C), or a combination of DAR and DAM was used to independently aggregate FcεRI and p58.2 KIR *via* rat IgE and mouse GL183 respectively (Fig.4D). As shown in Fig. 4A (closed circles), FcεRI-p58.2 KIR co-aggregation induced a GL183 dose-dependent inhibition of FcεRI-induced serotonin release, consistent with the observations reported in Fig.3A. By contrast, the independent aggregation of FcεRI and p58.2 KIR failed to inhibit FcεRI-induced serotonin release at any GL183 concentration (Fig. 4A, open circles). Similar results were obtained when RTIIB.p58 serotonin release was induced *via* the CD25/CD3ζ chimeric molecule (Fig.4B). These results demonstrate that KIR require a co-aggregation with activatory receptors (FcεRI or CD25/CD3ζ), in order to exert their inhibitory function. They also suggest that KIR-mediated inhibition takes place in the immediate vicinity of KIR molecules. Consistent with this conclusion, co-aggregation of FcεRI and KIR only inhibits RTIIB cell serotonin release induced by FcεRI but not by triggering of the CD25/CD3ζ chimeric molecule.

An inhibitory effect of p58.2 KIR should therefore only be observed when the relative proportion of co-aggregated KIR-activatory receptors is high enough.

To test this point, a clone expressing an unusually high level of CD25/CD3ζ chimeric molecules was selected. In this particular clone, using saturating concentration of GL183 F(ab')₂ (1 µg/ml), only low levels of inhibition (14.1% ± 5.0) of CD25/CD3ζ-induced serotonin release were observed.

In addition, it has been observed that in all clones stimulated with supra-optimal IgE concentrations (>10⁻³), KIR fail to inhibit FcεRI-induced RTIIB serotonin release. These data confirm that an inhibitory effect requires the co-aggregation of activatory and inhibitory receptors.

***KIR-FcεRI co-aggregation inhibits FcεRI-induced intracytoplasmic Ca***^{***2+***} ***mobilization**.* RTIIB serotonin release mediated *via* ITAM triggers Ca²⁺ᵢ mobilization. In order to test whether reconstituted human KIR inhibit early ITAM-dependent activatory signals, measurements of Ca²⁺ᵢ were performed using a single cell imaging system.

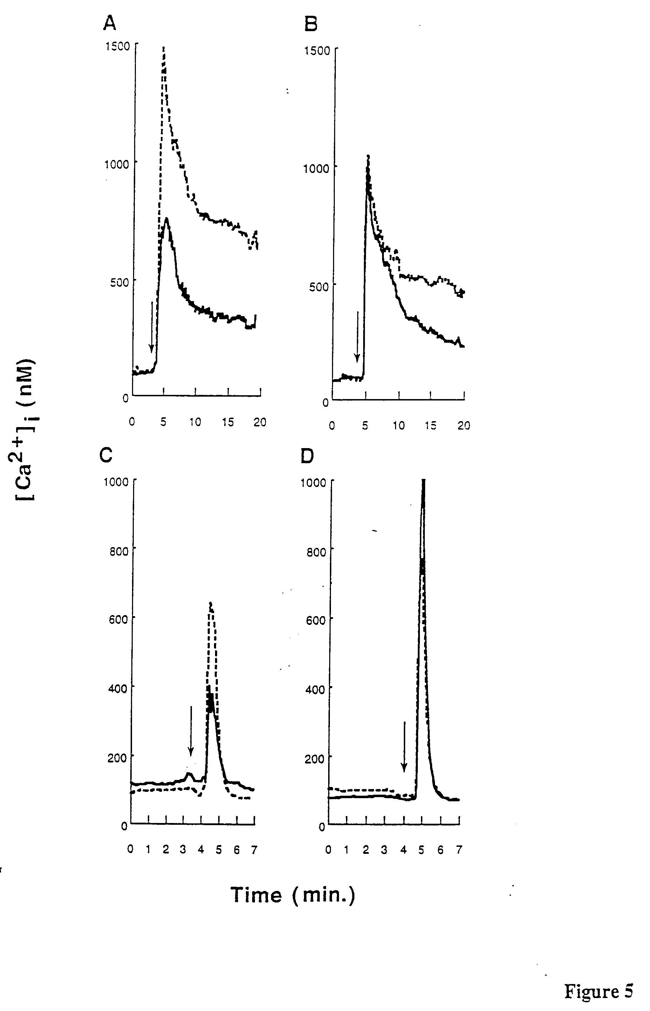

Results are reported in figure 5: Dotted line: RTIIB.p58 cells were pre-incubated with mouse IgE mAb (2682-I) (10⁻³ dilution). Continuous lines: RTIIB.p58 cells were pre-incubated 1 hour with a combination of mouse IgE mAb (10⁻³ dilution) and GL183 F(ab')₂ mouse mAb (1 µg/ml), (**A**,**C**) or mouse IgE mAb (10⁻³ dilution) and 2.4G2 F(ab')₂ rat mAb (1 µg/ml) (**B**,**D**). At a time indicated by the arrow, cells were stimulated with a GAM F(ab')₂ (50 µg/ml) (**A**,**C**), or TNP-F(ab')₂MAR (10 µg/ml) (**B**,**D**). (**A**,**B**): RTIIB.p58 cells were stimulated in the presence of extracellular calcium. (**C**,**D**) RTIIB.p58 cells were stimulated in the absence of extracellular calcium. Values were obtained from 59 to 117 tested cell from 3 to 5 independent experiments.

RTIIB.p58 and RTIIB.p50 cells were stimulated *via* the FcεRI receptor complex in the absence or presence of GL183 using GAM as a cross-linker. In both RTIIB.p58 and RTIIB.p50 cells, aggregation of the FcεRI receptor complex induced a large Ca²⁺ᵢ response consisting in a peak followed by a sustained plateau (Fig. 5A, dotted line). When p58.2 KIR was co-aggregated with FcεRI, the IgE-induced Ca²⁺ᵢ peak was impaired (Fig. 5A, continuous line). Using saturating concentrations of both GL183 F(ab')₂ (1 µg/ml) and IgE (10⁻³ dilution), the Ca²⁺ᵢ reached at the peak decreased from 1459 nM ± 92 (n=58 cells) to 756 nM ± 59 (n=60 cells) (mean ± SEM, p<0.001) (see Table 1 below).

**Table I**

| **Control of Ca**^{**2+**}_{**i**} **mobilization in RTIIB, RTIIB.p50 and RTIIB.p58 cells** | | | | |
|---|---|---|---|---|
| | IgE + GAM | IgE + GL183 + GAM | IgE + TNP-F(ab')₂MAR | IgE + 2.4G2 + TNP-F(ab')₂MAR |
| RTIIB | | | | |
| Basal Level ^{(a)} | 104 ± 5 | 104 ±3 | | |
| Peak response ^{(b)} | 1234 ± 78 | 1287 ± 68 | N.D.^{(f)} | N.D.^{(f)} |
| Plateau ^{(c)} | 645 ± 35 | 692 ± 36 | | |

| RTIIB.p50 | | | | |
|---|---|---|---|---|
| Basal Level ^{(a)} | 145 ± 5 | 165 ± 6 | | |
| Peak response ^{(b)} | 829 ± 67 | 873 ± 67 | N.D.^{(f)} | N.D.^{(f)} |
| Plateau ^{(c)} | 519 ± 26 | 534 ± 25 | | |

| RTIIB.p58 | | | | |
|---|---|---|---|---|
| Basal Level ^{(a)} | 110 ± 4 | 100 ± 5 | 102 ± 5 | 96 ± 4 |
| Peak response ^{(b)} | 1459 ± 92 | 756 ± 59 | 1032 ± 47 | 985 ± 48 |
| Plateau ^{(c)} | 700 ± 35 | 339 ± 25 | 515 ± 27 | 253 ± 12 |

| RTIIB.p58 without calcium ^{(e)} | | | | |
|---|---|---|---|---|
| Basal Level ^{(a)} | 99 ± 3 | 118 ± 5 | 104 ± 7 | 79 ± 2 |
| Peak response ^{(b)} | 625 ± 39 | 368 ± 32 | 712 ± 32 | 901 ± 36 |
| Plateau ^{(c)} | 67 ± 3 | 94 ± 6 | 74 ± 4 | 66 ± 3 |
| IgE (2682-I), GL183 F(ab')₂, 2.4G2 F(ab')₂, GAM F(ab')₂, and TNP-MAR F(ab')₂ were used as indicated in Fig. 5. | | | | |

| | | | | |
|---|---|---|---|---|
| a) [Ca²⁺]ᵢ at 2 minutes, before stimulation in nM ± SEM. | | | | |
| b) [Ca²⁺]ᵢ at the peak of the response in nM ± SEM. | | | | |
| c) [Ca²⁺]ᵢ at 17 minutes in nM ± SEM. | | | | |
| d) [Ca²⁺]ᵢ at 12 minutes in nM ± SEM. | | | | |
| e) the experiment was performed in the absence of extracellular calcium and in the presence of 0.5 mM EGTA as indicated in experimental procedures. | | | | |
| f) N.D.: Not done. | | | | |
| Values were obtained from 59 to 117 tested cells from 3 to 5 independent experiments. | | | | |

In contrast, when p50.2 KAR was co-aggregated with FcεRI, no significant alteration of IgE-induced Ca²⁺ᵢ mobilization was observed. At the Ca²⁺ᵢ peak, using saturating concentrations of both GL183 (1 µg/ml) and IgE (10⁻³ dilution), Ca²⁺ᵢ was 829 nM ± 67 (n=67 cells) *vs*. 873 nM ± 67 (n=67 cells) (mean ± SEM) for IgE *vs.* IgE-GL183 co-aggregation respectively (Table 1).

In order to evaluate whether KIR inhibited ITAM-dependent release of Ca²⁺ᵢ from intracellular stores and/or Ca²⁺ᵢ influx, further experiments were performed on RTIIB.p58 cells in the absence of extracellular Ca²⁺. In these conditions, only a small peak corresponding to the release of Ca²⁺ from intracellular stores was observed upon IgE stimulation (Fig. 5C, dotted line). Using saturating concentrations of both GL183 (1 µg/ml) and IgE (10⁻³ dilution), Ca²⁺ᵢ decreased from 625 nM ± 39 (n=56 cells) to 368 nM ± 32 (n=37 cells) (mean ± SEM, p<0.001) at the Ca²⁺ᵢ peak (see Table 1 above).

Therefore, p58.2 KIR is able to inhibit the release of Ca²⁺ from intracellular stores upon co-aggregation with FcεRI. In addition, no Ca²⁺ᵢ mobilization was detected when p58.2 or p50.2 were aggregated on RTIIB.p58 and RTIIB.p50 cells in the absence of IgE stimulation.

These results show that p58.2 KIR impairs ITAM-induced Ca²⁺ mobilization in RTIIB cells. Furthermore, p50.2 KAR was not capable of mediating any detectable Ca²⁺ mobilisation when expressed in RTIIB cells, in contrast to its stimulatory function reported in both T and NK cells.

### FcγRIIB-FcεRI co-aggregation inhibits extracellular Ca²⁺ influx in RTIIB cells, but does not inhibit intracellular Ca²⁺ mobilization.

RTIIB.p58 cells also express FcγRIIB ITIM-bearing receptor. Similar to KIR, FcγRIIB inhibit serotonin release in RTIIB cells. But by contrast to KIR, FcγRIIB only inhibit Ca²⁺ entry in B cells. Therefore, the effect of FcγRIIB-FcεRI co-aggregation on Ca²⁺ mobilization was examined, in order to state whether the differential effect of KIR and FcγRIIB on Ca²⁺ mobilization is due to a difference between B cells and RTIIB cells or is the consequence of distinct inhibitory strategies used by these two ITIM-bearing receptors.

RTIIB.p58 cells pre-incubated with IgE (10⁻³ dilution) in the presence or absence of 2.4G2 F(ab')₂ (1 µg/ml), were challenged with a TNP-F(ab')₂MAR (10 µg/ml). Aggregation of the FcεRI receptor complex induced a large Ca²⁺ᵢ response consisting in a peak followed by a sustained plateau (Fig. 5B, dotted line). When FcγRIIB was co-aggregated with FcεRI, the Ca²⁺ᵢ peak was not impaired but the plateau was not sustained (Fig. 5B, continuous line). Using saturating concentrations of both 2.4G2 F(ab')₂ (1 µg/ml) and IgE (10⁻³ dilution), the Ca²⁺ᵢ reached at the plateau decreased from 515 nM ± 27 (n=60 cells) to 253 nM ± 12 (n=59 cells) (mean ± SEM, p<0.001) (see Table 1 above).

In order to dissect the effects of FcγRIIB inhibition on Ca²⁺ mobilization, further experiments were performed on RTIIB.p58 cells in the absence of extracellular Ca²⁺. In these conditions, only a peak corresponding to the release of Ca²⁺ from intracellular stores was observed upon IgE stimulation (Fig. 5D, dotted line). Using saturating concentrations of both 2.4G2 (1 µg/ml) and IgE (10⁻³ dilution), no inhibition of Ca²⁺ release was observed, but rather Ca²⁺ᵢ increased from 712 nM ± 32 (n=79 cells) to 901 nM ± 36 (n=59 cells) (mean ± SEM) at the Ca²⁺ᵢ peak (Table 1 above and Fig. 5D). These results indicate that FcγRIIB has no effect on the release of Ca²⁺ from intracellular stores upon co-aggregation with FcεRI. In addition, no Ca²⁺ᵢ mobilization was detected when FcγRIIB was aggregated on RTIIB.p58 cells in the absence of IgE stimulation.

Therefore p58.2 KIR and FcγRIIB ITIM-bearing receptors exert distinct regulatory function on FcεRI-dependent Ca²⁺ mobilization.

### Discussion

HLA-Cw3-specific human KIR expressed in RTIIB cells can therefore inhibit Ca²⁺ᵢ mobilization and serotonin release induced by the FcεRI receptor as well as by a CD25/CD3ζ chimeric molecule (Fig 3, Fig.5). Therefore, KIR and FcγRIIB share several features.

First, KIR and FcγRIIB inhibit early steps of the signaling cascade, which are transcription-independent and are likely to reflect NK cell killing mechanisms, such as regulated exocytosis.

Second, KIR and FcγRIIB control the signals induced *via* polypeptides including only one ITAM (FcεRI), as well as receptors including three sequential ITAM (CD3ζ).

However KIR and FcγRIIB appear to use distinct inhibitory strategies. Indeed KIR inhibits Ca²⁺ᵢ release from ER stores whereas FcγRIIB only inhibit influx from the extracellular compartment. In addition, upon phosphorylation of the ITIM, FcγRIIB recruits preferentially the phosphatidyl inositide 5-phosphatase SHIP, whereas KIR recruits the SHP-1 tyrosine phosphatase (see Example 2). These two findings could be related.

These results also provide new insights on the importance of the cellular environment for the functions of a novel receptor family, characterized either by intact intracytoplasmic ITIM (FcγRIIB and KIR), or by their mutated version (KAR).

Finally, it is herein demonstrated that the mere cell surface expression of KIR does not modulate RTIIB cell activation in contrast to other regulators of lymphocyte activation, such as CD45.

On the contrary, co-aggregation between an activatory receptor (the FcεRI receptor complex or the CD25/CD3ζ chimeric molecule) and p58.2 KIR is required for the inhibition of serotonin release by RTIIB cells (Fig.4). This implies that the KIR inhibitory effect occurs in the immediate vicinity of the molecule.

The absolute requirement for a co-engagement of KIR with an activatory receptor may reflect the necessity for KIR to be phosphorylated by a non diffusible PTK associated with the activatory receptor. Indeed, the tyrosine phosphorylation of KIR is mandatory to the recruitment of SHP-1. Thus, the same PTK might induce the tyrosine phosphorylation of both ITAM and ITIM, which is consistent with data showing that ITIM YxxL/V sequence is a potential substrate for the src-family member PTK used by ITAM-containing receptor, such as lyn, lck an fyn. An additional basis for the obligation of proximity between activatory and inhibitory molecules may be that the tyrosine phosphorylated targets of SHP-1 likely belong to the ITAM-dependent activation cascade, and must be thus brought in close proximity to SHP-1.

The identification of the major targets for SHP-1 involved in the KIR inhibitory pathways is not yet achieved. Nevertheless, the recognition of target cells protected from NK cell lysis by the surface expression of HLA-B alleles leads to an inhibition of phosphatidyl inositol 4,5 biphosphate hydrolysis, resulting in the prevention of Ca²⁺ᵢ mobilization in NK cells. Similarly, it is herein demonstrated that KIR inhibit ITAM-induced Ca²⁺ᵢ mobilization in RTIIB cells. Therefore, phospholipase C-γ, and/or its potential upstream signaling effector/adaptors, such as p36-38, as well as the ITAM-binding SH2-tandem PTK (ZAP-70 and p72^{Syk}) may be SHP-1 targets involved in KIR signaling pathways.

In conclusion, the present invention allows to define and extend the family of ITIM-bearing receptors, involved in the negative control of cell activation: the T cell-specific CD3/TCR complex pathway and the KIR pathway, both regulating T and NK cell cytotoxicity, are complementary, and permit to eliminate a cell presenting a foreign antigen in the context of MHC Class I molecules as well as a cell expressing no (or a modified form of) MHC Class I molecules.

T lymphocytes, the activation of which KIR can control, are involved in the control of potential autoimmune reactions as well as other inflammatory/immune reactions which may be deleterious. In addition, expression of KIR during viral infection, may overcome a deficient CD3/TCR triggering.

Indeed various virus interfere with assembly and transport of Class I molecules to the cell surface, which might result in a less efficient presentation and/or expression of structurally abnormal Class I molecules. As a result, CTL will be less efficiently stimulated by the CD3/TCR complexes, but more efficiently stimulated because of the absence of KIR engagement by MHC Class I molecules.

The above-reported findings therefore demonstrate that the threshold of T cell activation depends not only on the TCR-ligand avidity and the number of TCR engaged, but also depends on the engagement or the non-engagement of KIR. Several alternative pathways are utilized in T cells to control the activation programs, and similarly to KIR these pathways appear to act on the early PTK-dependent steps of T cell activation.

### Example 2: In contrast to FcγRIIB, KIR do not bind SHIP and recruit SHP-1 and SHP-2.

### Obtention of an antiserum specifically recognizing the tyrosine phosphorylated but not the non-phosphorylated form of both the N-and C-terminal KIR ITIMs.

### Experimental procedures :

**Peptides and antisera**. The following peptides were synthesized as phosphorylated or not, and contain an N-terminal-biotin :

| **PEPTIDE** | **SEQUENCE** |
|---|---|
| p58.2.1 (N-terminal ITIM) : | DEQDPQEVT**Y**_{**303**}AQLNH |
| p58.2.1-V³⁰¹A : | DEQDPQEAT**Y**_{**303**}AQLNH |
| p58.2.2 (C-terminal ITIM) | RPSQRPKTPPTDIIV**Y**_{**333**}TELPNAEP |
| FcγRIIB : | KTEAENTIT**Y**_{**262**}SLLK |
| FcγRIIB-I₂₆₀A : | KTEAENTAT**Y**_{**262**}SLLK |
| CD3ξ1 : | YQGQNQL**Y**_{**71**} NELNLGRREE**Y**_{**82**}DVLDKRRGR |

The p58.2 peptides correspond to the human p58.2 KIR sequence. The FcγRIIB peptides correspond to the murine FcγRIIB1/B2 sequence which is highly homologous to the human sequence : KVGAENTITYSLLM. The murine sequence was chosen because of the impossibility at generating phosphopeptides corresponding to the human sequence. The 712 rabbit antiserum was generated using phosphorylated p58.2.1 peptide coupled to ovalbumine as an immunogen (Neosystem).

**Fusion proteins and surface plasmon resonance analysis**. Surface plasmon resonance (SPR) measurements were performed on a BIAcore™ (Pharmacia). The GST-SHP-1.SH2(NC), GST-SHP-1.SH2(N), GST-SHP-1.SH2(C), GST-SHIP.SH2 fusion proteins generated from the murine phosphatase cDNA, were purified from DH5α lysates. Briefly, overnight culture at 37°C in LB medium containing 50 µg/ml ampicillin was diluted 1/10 in fresh medium, and incubated until the absorbance at 600 nm reaches 1-2. At that point, IPTG was added (1mM), and incubation continued for an additional 4 hours at 37°C. After centrifugation, bacteria pellet was resuspended in TENGN buffer (50 mM Tris pH 8,1 mM EDTA, 10 % glycerol, 1 % NP40, 1 mM DTT, 1 mM PMSF, 10 µg/ml aprotinin and 10 µg/ml leupeptine). Bacteria were lysed by sonication. After centrifugation, supernatant was incubated with Glutathione-Sepharose 4B beads (Pharmacia) overnight at 4°C with slow shaking. After 3 washes with 50 mM Tris pH 8, elution of the fusion protein was carried out using 50 mM Tris pH 8 supplemented with 10 mM reduced glutathione. Before their use in BIAcore™ experiments, fusion proteins were dialyzed in HBS buffer (10 mM HEPES, 150 mM NaCl, 3.4 mM EDTA). Protein purity was assessed by 12.5 % SDS-PAGE, and Coomassie blue staining. The running buffer used in all BIAcore™ experiments consisted of HBS buffer supplemented with 0.05 % surfactant P20. For preparation of microchips coated with phosphorylated peptides, immunopure streptavidine (Sigma) was first immobilized onto CM5-sensorchip. Then, biotinylated peptides were injected, and their binding to streptavidine-coated microchips was assessed using anti-phosphotyrosine 4G10 mAb (UBI).

**Assay for cell lysate adsorption to peptides**. RBL-2H3 cells were lysed in NP-40 lysis buffer (1 % NP-40, 10 mM Tris-HCl, 150 mM NaCl, 1 mM EDTA, 1 mM PMSF, 10 mM iodoacetamide, 10 mM NaF, 10 mM Na pyrophosphate, 0.4 mM Na vanadate, 10 µg/ml leupeptine, 10 µg/ml aprotinine). Samples were either used directly (whole cell lysates : WCL), or subjected to affinity purification using peptides bound to beads. Biotinylated peptides (5 µg) were coupled to 50 µl streptavidin-agarose bead slurry (Sigma) for 1 hour at 4°C, prior to bead saturation with D-biotin (1 mg/ml) for 1 hour at 4°C. After 3 washes in lysis buffer, samples were separated on SDS-PAGE and transferred to nitrocellulose. Immunoblots were revealed using anti-SHP-1, anti-SHP-2 mAb (0.5 µg/ml) (Transduction laboratories) or anti-SHIP antiserum and ECL (Amersham).

### Cell activation and immunoblotting.

Cells from a representative clone of each transfected cell line, were washed 3 times in cold PBS, resuspended at 3 x 10⁶ cells/ml in cold PBS and incubated for 30 min at 4°C in the presence or absence of the indicated purified mAb (5 µg/ml). After 1 wash in PBS, cells were resuspended at the same concentration in the presence of 5 µg/ml F(ab')₂ goat anti-mouse antiserum (GAM, Immunotech) for 3 min at 37°C. Cells were then instantly lysed in NP-40 lysis buffer for 15 min on ice. After removing insoluble material by centrifugation at 12,000 rpm for 15 min, samples were either used directly or subjected to immunoprecipitation for 2 hr using GL183 covalently coupled to CnBr-beads (Pharmacia). Samples were then combined with reducing sample buffer (New England Biolabs) and boiled, before separation on 10 % SDS-polyacrylamide gel electrophoresis (SDS-PAGE). Immunoblots using 712 were revealed using horseradish peroxydase-conjugated goat anti-rabbit antiserum (Sigma) and ECL detection system (Amersham).

### Results and discussion :

In order to dissect the signaling pathways used by KIR, we first investigated whether the co-aggregation between the HLA-Cw3-specific p58.2 KIR, and an ITAM-bearing receptor, FcεRI, could modulate the tyrosine phosphorylation of KIR ITIM *in vivo*.
In this regard, antisera directed towards the tyrosine phosphorylated form of KIR ITIM peptides were generated. Among this series, antiserum 712 specifically recognizes the tyrosine phosphorylated-, but not the non-phosphorylated form of both the N- and the C-terminal KIR ITIMs.
The 712 antiserum was further used to probe whole cell lysates as well as anti-KIR immunoprecipitations prepared from stimulated and unstimulated reconstituted KIR⁺ cells.
RBL-2H3 cell transfectants which express endogenous FcεRI and exogenous p58.2 KIR (RTIIB. p58 cells), were stimulated using anti-FcεRI (BC4), anti-p58.2 KIR (GL183) mAbs alone or in combination, in the presence of GAM as a cross-linker.
Although tyrosine phosphorylation of KIR was detected in whole cell lysates upon anti-p58.2 KIR cross-linking, a major increase in KIR tyrosine phosphorylation was observed when p58.2 KIR was co-aggregated with the FCεRI ITAM-containing complex.
KIR contain only 2 intracytoplasmic tyrosine residues which are included in the N- and C-terminal ITIMs respectively. Therefore, reactivity of the 712 anti-KIR ITIM antiserum can only account for KIR ITIM tyrosine phosphorylation. These results were confirmed using 712 immunoblotting of anti-p58.2 immunopre-cipitations. As a control, no tyrosine phosphorylation of KIR was detected when cell lysates were prepared from the RTIIB.p50 cell transfectants. RTIIB.50 cells express p50.2, a naturally-occurring mutant of p58.2, that contains a truncated form of p58.2 intracytoplasmic domain which is devoid of both ITIMs and inhibitory function.
These results indicate that co-aggregation between KIRs and ITAM-bearing receptors greatly enhances the tyrosine phosphorylation of KIR ITIMs, consistent with its requirement for KIR inhibitory function. However, co-aggregation of KIR with FcεRI is not mandatory to KIR tyrosine phosphorylation, in agreement with the reported association of KIRs with the p56^{lck} PTK.
Therefore, co-aggregation between KIRs and ITAM-bearing receptors may be also required at a later step than KIR tyrosine phosphorylation. Indeed, phosphorylated forms of KIR ITIM recruit SHP-1 and SHP-2 PTPases suggesting that co-aggregation is required for bringing the PTPases in the close vicinity of their tyrosine phosphorylated substrates, which likely belong to the ITAM-dependent cascade. In this regard, the SH2-tandem PTK, ZAP-70 has been recently shown to be a direct substrate of SHP-1.

In constrast to KIR, FcγRIIB cross-linking in RBL-2H3 cell transfectants does not lead to any detectable FcγRIIB ITIM tyrosine phosphorylation, suggesting that these two distinct ITIM-bearing negative coreceptors use diverse strategies in order to mediate their inhibitory function.

Since the inositol polyphosphate 5-phosphate, SHIP and the phosphatases SHP-1, SHP-2 are involved in FcγRIIB inhibitory function, the ability of both KIR N- and C-terminal ITIMs to bind SHIP *in vitro* was investigated.

Whereas both phosphorylated forms of KIR ITIM bind SHP-1 and SHP-2, no binding to SHIP was detected. As a control, only the tyrosine phosphorylated form of FcγRIIB ITIM recruit SHIP, SHP-1 and SHP-2 phosphatases. The recombinant GST-fusion protein and SPR analysis confirmed that the phosphorylated form of KIR ITIM does not associate with SHIP, in contrast to FcγRIIB ITIM. The absence of direct SHIP binding *in vitro* to KIR ITIM is consistent with the absence of SHIP recruitment to KIR *in vivo*, and is likely to have physiological implications. Indeed we have previously observed (see example 1) that in the same RTIIB.p58 cells, which express both FcγRIIB and p58.2 KIR, the mechanisms used by both ITIM-bearing receptors to inhibit FcεRI-induced cell activation are divergent: whereas KIR and FcεRI co-aggregation leads to inhibition of Ca²⁺ release from the endoplasmic reticulum, FcγRIIB and FcεRI co-aggregation leads to the inhibition of Ca²⁺ influx form the extracellular milieu.

The differential recruitment of PTPases or SHIP by KIR and FcγRIIB may therefore be involved in the differential effect of both ITIM-bearing negative coreceptors on Ca²⁺ mobilization. In this regard, SHIP is a polyphosphate inositol 5-phosphatase which remains to be characterized for its function as a regulator of phosphatidyl-inosotol 4,5 biphosphate and inositol 1,4,5 triphosphate metabolism.

Irrelevant of their differential binding properties, KIR and FcγRIIB ITIM share a common isoleucine or valine residues at a position tyrosine-2. Similarly, another hematopoietic ITIM-bearing negative coreceptor gp49B1, which is expressed on mast cells and inhibits FcεRI-mediated activation, also contains isoleucine and valine residues at the position tyrosine-2 in its two ITIMs respectively.

Phosphorylated KIR and FcγRIIB ITIM peptides corresponding to a single point aminoacid substitution, *i.e.* p58.2.1-V₃₀₁A and FcγRIIB 1-I₂₆₀A, were generated. These peptides were tested for their ability to bind SHP-1, SHP-2, and SHIP phosphatases in a cell lysate adsorption assay, in comparison to the wild type KIR and FcγRIIB ITIM peptides. Substitution of isoleucine and valine by an alanine residue abolishes the binding of SHP-1 and SHP-2 to both KIR and FcγRIIB ITIM peptides.

In contrast, binding of SHIP to mutated FcγRIIB ITIM is not affected. Similar results were obtained using SPR for SHP-1 and SHIP, confirming that the tyrosine-2 amino-acid position in ITIMs is critical for binding to PTPase but not to SHIP.

Since KIRs do not associate with SHIP, we also further characterized the interaction between KIRs and SHP-1, which is likely to be involved in the inhibitory function of KIRs. We thus determined using SPR the kinetic constants of the interaction between KIR ITIM peptides and recombinant fusion proteins corresponding to the isolated N- and C-SH2 domains of SHP-1. Both the isolated SHP-1.SH2 domains bind the phosphorylated form of KIR ITIMs, in contrast to previous results reporting that only the C-SH2 domain was responsible of the interaction between KIR and SHP-1.

In the following table 2, are shown the association and dissociation constants for the interaction of isolated SHP-1.SH2(N) and SHP-1.SH2(C) domains with phosphorylated KIR ITIM peptides :

**Table 2**

| Phosphorylated peptides | GST-SHP-1.SH2(N) | | | GST-SHP-1.SH2(C) | | |
|---|---|---|---|---|---|---|
| | *k*ₒₙ (10⁻⁴M⁻¹s⁻¹) | *k*_{off} (10³ s⁻¹) | *K*_{d} (nM) | *k*ₒₙ (10⁻⁴M⁻¹s⁻¹) | *k*_{off} (10³ s⁻¹) | *K*_{d} (nM) |
| p58.2.1 | 10.0 | 42 | 423 | 0.50 | 0.7 | 137 |
| p58.2.2 | 0.8 | 25 | 3160 | 0.06 | 0.3 | 545 |

The measurement of phosphorylated p58.2 peptide binding to GST-SHP-1.SH2(N) and GST-SHP-1.SH2(C) fusion proteins was at a constant 5 µl/min flow rate. In the representative experiment of which results are reported in the above table I, 130 and 160 RU of respectively p58.2.1 and p58.2.2 peptides were immobilized on microchips. The regeneration was performed using HBS buffer supplemented with 0.03 % SDS. Results are expressed as corrected resonance units (CRU) corresponding to the raw RU values subtracted from background RU value due to the injection medium. In this representative experiment, 130 RU of p58.2.1 peptide and 160 RU of p58.2.2 peptide were immobilized on microchips. *k*_{off} and *k*ₒₙ were calculated from three independent measurements using the BIAevaluation 2.0 software. *K*d was calculated from *k*_{off}/*k*ₒₙ.

However, striking differences were observed between the binding capacities of the isolated SH2 domains. Measurement of kinetic constants revealed that the affinity of the phosphorylated N-terminal KIR ITIM peptide for SHP-1.SH2(C) is 3-3.5 times higher than for SHP-1.SH2(N) (see Table 2). This difference is the direct consequence of a dramatically higher *k*_{*off*}*,,* despite an higher k_{*on*}*,,*in the interaction of the phosphorylated KIR ITIM peptide with SHP-1.SH2(N) as compared to SHP-1.SH2(C). The N- and C-SH2 domains of SHP-1 exert distinct regulatory roles on SHP-1 : whereas the C-H2 domain merely acts as a recruiting unit, the N-SH2 domain not only serves as a docking unit but also as a regulator for SHP-1 PTPase activity.

Therefore, our results showing that KIRs associate with both SHP-1 N- and C-SH2 domains, are in agreement with their reported role as activators of SHP-1 PTPase function. These data also confirm that the N-terminal KIR ITIM bind SHP-1.SH2(N) and SHP-1.SH2(C) domains more efficiently than the C-terminal KIR ITIM, and support the observation showing that the N-terminal KIR ITIM is sufficient for mediating KIR inhibitory function.

Nevertherless, the binding of both KIR ITIMs to SHP-1 SH2 domains, is reminiscent of the association between SHP-2 SH2 domains and two distinct IRS-1 amino-acid stretches surrounding tyrosine residues 1172 and 1222. The crystallographic analysis of SHP-2 SH2 domain structure revealed that the distance between tyrosine 1172 and tyrosine 1222 is critical for the simultaneous association of both IRS-1 binding sites to SHP-2.SH2(N) and SHP-2.SH2 (C), which leads to a dramatic increase in SHP-2 PTPase activity. Since the tyrosine residues present in the N- and C-terminal KIR ITIMS are separated by 30 amino-acids (tyrosine 303 and tyrosine 333 respectively), it is possible that this distance may be sufficient to allow a simultaneous binding of both KIR ITIMs to the N- and C-SH2 domains of SHP-1 and SHP-2 PTPases.

In summary, our data contribute to define the structure of ITIMs, in which the position tyrosine-2 appears to be critical for the binding of SH2-containing PTPases, but not for the binding of SHIP. They also reveal that ITIM-bearing negative coreceptors recruit distinct sets of SH2-containing phosphatases and use divergent strategies in order to mediate their inhibitory function.

### Example 3: The human lectin-like NKG2A/B KIR heterodimer recruits the phosphatase SHP-1

The human CD94-NKG2A/B KIR heterodimer is a lectin-like HLA Class I receptor. In this set of experiments, cells isolated from patients presenting a Lymphoproliferative Disease of Granular Lymphocytes (LDGL) of the CD3- natural killer (NK) cell lineage were used. These cells expressed no detectable human Ig-like KIR but expressed CD94 and NKG2A at their surface as assessed by flow cytometric analysis. Cells (5 x 10⁶/sample) were incubated for 5 minutes at 37°C in the presence or absence of 200 µM pervanadate. After incubation, cells were instantly lysed at 4°C in NP-40 lysis buffer. Post-nuclear supernatants were then immunoprecipitated using mouse monoclonal antibodies directed against human CD94 (XA-185), human NKG2A/B (Z199) or rat FcεRIα (BC4). Samples were separated on SDS-PAGE, and probed for the presence of SHP-1 by immunoblot using a mouse mAb directed towards SHP-1. These experiments reveal that the human CD94-NKG2A/B KIR heterodimer recruits SHP-1. This is the first demonstration of a human lectin-like KIR recruitment of a phosphatase.

### Example 4: the KIR homologue gp49B1 can bind SHP-1, SHP-2 and SHIP, differential recruitment of SHIP and of SHP-1 by gp49B1 peptides and inhibition of SHP-1 binding on phosphorylated p58.2 peptide by gp49B1 peptides.

Peptides corresponding to the intracytoplasmic gp49B1 ITIM-like sequences were synthesized in a tyrosine-phosphorylated as well as in a non-phosphorylated form. The gp49B1 N-term is a peptide which corresponds to the N-terminal gp49B1 ITIM, whereas the gp49B1 C-term is a peptide which corresponds to the C-terminal gp49B1 ITIM.

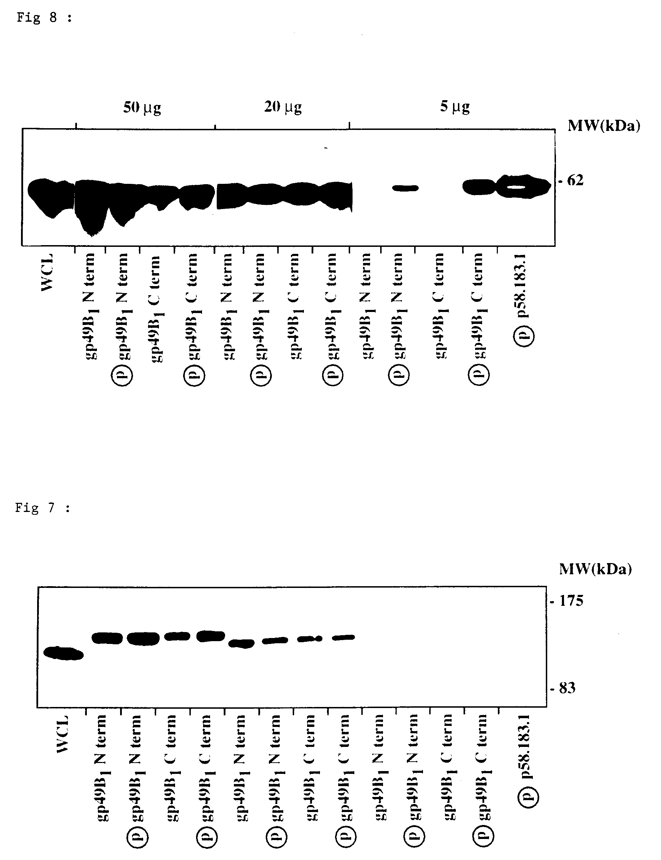

Peptides as indicated on figures 7 and 8 were immobilized on CnBR-beads (Pharmacia). RBL-2H3 cells were lysed without any stimulation in NP-40 lysis buffer, and post-nuclear cell lysates (proteins) were allowed to adsorb on indicated quantities of peptide-coated beads for 3hr at 4°C. The beads were then washed 3 times in NP-40 lysis buffer and probed for the presence of SHP-1 or SHIP using specific anti-SHP-1 and anti-SHIP antibodies and immunoblotting.

Results are reported on figures 7 and 8 where the differential recruitments of SHP-1 and of SHIP by gp49B1 peptides are illustrated by Western blots of phosphorylated (P) or non-phosphorylated ( ) n-terminal (N-term) or C-terminal (C-term) gp49B1 peptides at different (5, 20, 50 µg) of cellular lysate proteins.
For the binding of the unphosphorylated forms, no binding was found for 500 µM and less. Finally, the percentage of inhibition of the phosphorylated p58.2.1 peptide on itself was 70 % at 7.5 µM.

2 ng of phosphorylated p58.2 N-terminal ITIM peptide were then immobilized on streptavidin microchips. Inhibition percentages were assessed by surface plasmon analysis after incubation of indicated concentration of gp49B1 peptides for 15 min at room temperature with GST-PTP1C-SH2 (N+C) at 500 nM, and injection over immobilized phosphorylated p58.2 N-terminal ITIM surface.

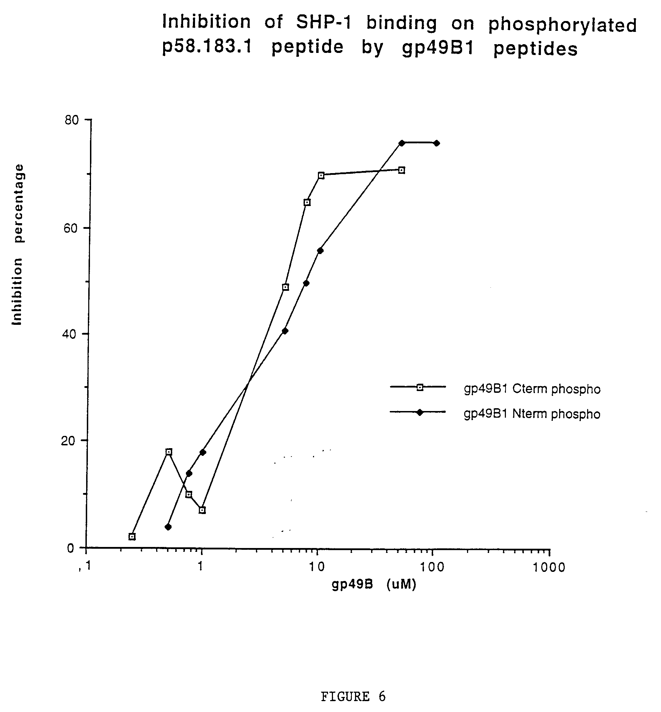

Results are reported on figure 6, showing the inhibition percentage of SHP-1 binding on phosphorylated p58.2 peptide as a function of gp49B1 concentration (µM).

Data reported in figure 6,7 and 8 document for the first time the in vitro binding of gp49B1 ITIM to SHP-1 (figures 6 and 8) and SHIP (figure 7). Non-specific binding of SHIP and SHP-1 to the non-phosphorylated forms of gp49B1 ITIM peptides are observed at 20 and 50 µg of peptides. At 5 µg of peptides, a specific binding of SHP-1 is observed onto the phosphorylated forms of gp49B1 ITIMs only. This result was confirmed using surface plasmon resonance (figure 6).

*In vivo* recruitment of SHIP to gp49B1 was obtained using bone-marrow derived mast cells (data not shown).

This demonstrates that the gp49B1 inhibitory receptor contains, in its intracytoplasmic domain, two ITIM-consensus motifs which behave as ITIMs (*i.e.* they bind phosphatase which are likely involved in the inhibitory transduction cascade). Therefore, gp49B1 is a member of the family of ITIM-bearing coreceptors.

### Example 7: Transgenic mice expressing a human KIR

Transgenic mice were generated using the cDNA encoding for p58.2 (cl.6), inserted in the HindIII version of the pHSE3' transgenic vector under the control of the H-2K^{b} promoter. Splenocytes and peripheral blood lymphocytes isolated from p58.2 transgenic animals were analyzed by immunofluorescence and flow cytometry.

These data reveal that the human p58.2 molecule is expressed at the cell surface of both mouse T and NK cells. The p58.2 Ig-like KIR recognizes the HLA-Cw3. Therefore, the cell surface expression of HLA-Cw3 confers the protection of target cells against NK-cell mediated natural cytotoxicity. Using the murine mastocytoma cells P815 transfected (P815-Cw3) or not with the HLA-Cw3 cDNA, it has been observed that NK cells isolated from p58.2 transgenic mice can induce the lysis of parental P815 cells but are inefficient in inducing the lysis of P815-Cw3 cells.

These data show for the first time the functional reconstitution of a human Ig-like KIR in the mouse model.

### Example 8 : Preparation of a bispecific diantibody capable of cross-linking a KIR with a stimulatory receptor in the intracytoplasmic domain

It has been shown (see example 3, antiserum 712) that rabbits can be immunized using synthetic p58.2 ITIM peptides. In these experiments, the ITIM peptides were coupled to ovalbumin. This data thus demonstrates that one can obtain specific anti-ITIM antibodies.

Using a similar immunization strategy, monoclonal antibodies directed against the intracytoplasmic domain of several ITIM-bearing molecules, including phosphorylated and non-phosphorylated KIR ITIMs can be generated.

Similarly, antibodies can be generated against the intracytoplasmic domains of ITAM-polypeptides included in the CD3/TCR, FcεRI as well as FcγIIIA receptor complexes.

In parallel, soluble fusion protein corresponding to the extracytoplasmic domain of KIRs can serve as immunogens to generate antibodies.

Diantibodies can therefore be generated from the above-mentioned antibodies by standard procedure.

A screening on the serotonin release of RBL-2H3 cell transfectants allows the selection of the most efficient compounds (diantibody, peptide, glycoprotein, carbohydrate).

## Claims

1. Compound capable of cross-linking a stimulatory receptor with a KIR or with an homologue thereof.

2. Compound according to claim 1, characterized in that it is capable of specifically regulating the activation of a KIR or a KIR homologue.

3. Compound according to claim 1 or 2, characterized in that it is capable of regulating the activation of a stimulatory receptor.

4. Compound according to any of the preceeding claims characterized in that said stimulatory receptor is an ITAM-bearing receptor such as KAR, FcεRI, CD3/TCR, CD16, receptors related to tyrosine kinase activities or a receptor sub-unit such as CD3ζ, CD3ε, CD3Υ, CD3δ or FcεRIγ.

5. Compound according to any one of claims 1-4, characterized in that said KIR is a IgSF member, particularly selected from the group comprising CD158, CDw159, CDw160, or said KIR is lectin-like, such as the CD94-NKG2A/B heterodimer, or is a KIR homologue, such as gp49B1.

6. Compound according to any of the preceeding claims, characterized in that said KIR or said KIR homologue is expressed on a NK cell, on a T cell, on a mast cell or on a monocyte or is recombinantly expressed.

7. Compound according to any of the preceeding claims, characterized in that it is capable of inducing the regulation of free calcium concentration in a cell, particularly of inducing the regulation of calcium influx into a cell and/or of inducing the regulation of calcium mobilization from intracellular compartments.

8. Compound according to any of the preceeding claims, characterized in that it is capable of inducing the recruitment by said KIR or KIR homologue of a phosphatase selected from the group consisting of SHP-1, SHP-2, SHIP and related phosphatases.

9. Compound according to any of the preceeding claims, characterized in that it is essentially a polypeptide, a glycoprotein or a carbohydrate.

10. Compound according to any of the preceeding claims, characterized in that said compound is a bispecific reagent and/or a chemical inducer of dimerization.

11. Compound according to any of the preceeding claims, characterized in that said compound is a bispecific antibody, comprising at least one Fab, Fd, Fv, dAb, CDR, F(ab')₂, VH, VL, ScFv fragment.

12. Compound according to any of the preceeding claims, characterized in that it is capable of cross-linking said KIR or said KIR homologue with said stimulatory receptor in the extracellular domain of a cell.

13. Compound according to any of the preceeding claims, characterized in that it is capable of crossing through a lipid bi-layer, and is liposoluble and/or associated with a drug-delivery system.

14. Compound according to any of the preceeding claims, characterized in that it is capable of cross-linking said KIR or said KIR homologue with said stimulatory receptor in the intracellular domain of a cell.

15. Compound according to any of the preceeding claims, characterized in that it is capable of modulating the release of serotonin and/or of inflammatory mediators by a cell expressing FcεRI, such as a mast cell, and/ or of modulating cytokine release, such as Interleukin-6, Tumor Necrosis Factor Alpha release, from a cell such as a mast cell or a NK cell and/or of modulating interleukin production such as the IL-2 production and/or the γ-interferon production from a peripheral blood cell and/or of modulating the proliferation of peripheral blood cells.

16. Nucleic acid coding for a polypeptide according to any one of claims 9-15.

17. Cell transfected by a nucleic acid according to claim 16.

18. Pharmaceutical preparation comprising a compound according to any one of claims 1-15 or a nucleic acid according to claim 16 or a cell according to claim 17 in a a physiologically acceptable vehicle, in a therapeutically-effective amount useful for modulating an animal cell function involved in a disease selected from the group consisting of immunoproliferative diseases, immunodeficiency diseases, cancers, autoimmune diseases, infectious diseases, viral diseases, inflammatory responses, allergic responses or involved in organ transplant tolerance.

19. Method for the *in vitro* or *ex vivo* diagnosis of a cell disregulation, comprising the step of estimating of the relative proportion of co-aggregated KIR or KIR homologue *vs*. non-co-aggregated KIR or KIR homologue by:
- contacting a biological sample with a compound according to any one of claims 1-15 or with a nucleic acid according to claim 16 or a cell according to claim 17, and
- revealing the reaction product possibly formed.
